# FASCICULE DE BREVET EUROPEEN

(11) **EP 1 871 399 B1**
(45) Date de publication et mention de la délivrance du brevet: **26.01.2011**
(21) Numéro de dépôt: 06743672.5
(22) Date de dépôt: 11.04.2006
(51) Int. Cl.: A61K 35/60, A61P 17/06

(54) **UTILISATION DE LA LECITHINE COMME MEDICAMENT DANS LE TRAITEMENT DU PSORIASIS**
VERWENDUNG VON LECITHIN ALS ARZNEIMITTEL ZUR BEHANDLUNG VON PSORIASIS
USE OF LECITHIN AS A MEDICAMENT FOR TREATING PSORIASIS

(30) Priorité: 18.04.2005 FR 0503827; 27.06.2005 FR 0506496
(43) Date de publication de la demande: 02.01.2008
(73) Titulaire: SC Dicophar, 31770 Colomiers (FR)
(72) Inventeur: DUPONT, Paul, F-31770 Colomiers (FR)
(74) Mandataire: Domange, Maxime
(86) Numéro de dépôt international: PCT/FR2006/000792
(87) Numéro de publication internationale: WO 2006/111633

(56) Documents cités:
- WO-A-98/42348
- WO-A-2005/002591
- US-A1- 2002 012 648
- ESCOBAR S O ET AL: "TOPICAL FISH OIL IN PSORIASIS-A CONTROLLED AND BLIND STUDY" CLINICAL AND EXPERIMENTAL DERMATOLOGY, BLACKWELL SCIENTIFIC PUBLICATIONS, OXFORD, GB, vol. 17, no. 3, 1992, pages 159-162, XP009030105 ISSN: 0307-6938
- MAYSER P ET AL: "[omega]-3 Fatty acid-based lipid infusion in patients with chronic plaque psoriasis: Results of a double-blind, randomized, placebo-controlled, multicenter trial" JOURNAL OF THE AMERICAN ACADEMY OF DERMATOLOGY 1998 UNITED STATES, vol. 38, no. 4, 1998, pages 539-547, XP002360544 ISSN: 0190-9622

## Description

La présente invention se rapporte à l'emploi de la lécithine pour la préparation d'une composition pharmaceutique utile dans la prévention et le traitement thérapeutique des dermatoses récentes ou anciennes, notamment du psoriasis.

Les formations squameuses et/ou kératosiques anormales du derme sont une des manifestations de l'état de la peau qui se rencontre dans de nombreuses dermatoses, qu'elles soient apparues de façon récentes ou installées de longue date, et en particulier dans les différentes formes du psoriasis.

Le psoriasis est une dermatose érythémo-squameuse d'évolution chronique, caractérisée par des plaques rouges recouvertes d'épaisses squames blanches, qui atteint environ 2% de la population. Avec plus de 60 000 nouveaux cas chaque année, c'est une des affections dermatologiques les plus fréquentes. Elle se manifeste par des démangeaisons douloureuses et des plaies pouvant atteindre plus de 10% du corps. Dans ses formes habituelles, sa localisation est très caractéristique : coudes, et bord cubital des avant-bras, genoux, région-lombo-sacrée, cuir chevelu, ongles. Des formes particulières, qualifiées de psoriasis graves, sont également connues, telles que le psoriasis érythrodermique, le psoriasis arthropathique ou le psoriasis pustuleux. Environ 1,5% des psoriasis débutent chez l'enfant avant l'âge de 10 ans et 35 % avant 20 ans. Cette anomalie du renouvellement de l'épiderme est donc une affection sérieuse représentant un problème de santé publique important

La cause du psoriasis est pour l'instant inconnue mais on considère que les facteurs impliqués sont multiples. Un facteur génétique a pu être mis en évidence, avec 30% de psoriasis familiaux, ainsi que des facteurs environnementaux. Les facteurs psychologiques peuvent fréquemment déclencher des poussées. Les traumatismes cutané tels que griffures, vaccinations, chirurgie, sont parfois le siège d'une efflorescence de lésions psoriasiques. L'alcool et le tabac sont des facteurs de gravité et de résistance thérapeutique.

L'évolution de la maladie est chronique et se fait par poussées généralement imprévisibles, entrecoupées de rémissions pendant lesquelles les lésions sont minimes. Même en dehors des formes graves, et bien que l'état général du malade ne soit pas altéré dans les formes habituelles, le psoriasis est une maladie qui peut perturber profondément la qualité de vie lorsque les lésions sont affichantes ou gênantes pour un travail manuel, conséquences qui sont souvent sous-estimées par le médecin.

A l'heure actuelle, les médecins disposent de traitements symptomatiques aux résultats transitoires, mais pas encore de médicaments curatifs.

Les traitements locaux, sous forme de pommade ou de crème, sont des lotions à base de cortisone et les dérivés de la vitamine D, ces deux produits étant souvent combinés. Les rétinoïdes (dérivés de la vitamine A) peuvent également être utilisés. Ils sont très utiles mais ne peuvent être correctement employés que si le psoriasis n'est pas trop étendu ou évolue peu. S'il dépasse 10% de la surface corporelle, près de la moitié des patients arrêtent de suivre ces traitements contraignants au bout d'un mois, faute de temps. En outre les dérivés de la vitamine D ont un potentiel irritant, et surtout les corticoïdes peuvent provoquer une atrophie cutanée et entraîner des rebonds de la maladie.

Les séances de photothérapie sont efficaces chez un grand nombre de patients. Elles consistent en une irradiation en cabine médicale par des rayons UVB ou UVA, en association avec des médicaments photosensibilisants. Les cures sont contraignantes, car les patients doivent se soumettre à trois séances hebdomadaires de deux heures pendant 8 à 10 semaines. Cette technique présente surtout l'inconvénient majeur d'une induction à long terme de cancers cutanés, ainsi que d'un vieillissement accéléré de la peau. Il est donc indispensable de la réaliser selon des règles très strictes.

Pour les patients qui n'ont pas été soulagés par les traitements locaux ou par la photothérapie, soit 30 % à 40% des malades, des traitements généraux sont proposés. Un de ces traitements repose sur la prise de rétinoïdes par voie orale, le plus souvent en association avec la photothérapie ou bien avec la prise de méthotréxate (Novatrex®) qui est le médicament de référence. Ce dernier ayant des effets secondaires indésirables, notamment hématologiques et hépatiques, sa prescription nécessite une surveillance étroite. La ciclosporine (Néoral ®) est également très efficace, mais du fait de sa toxicité rénale, elle ne peut pas être préconisée pour des traitements prolongés.

On comprend donc que les traitements préconisés jusqu'à présent ont tous des limitations importantes quant à leur prescription et à leur tolérance, et que leur efficacité reste incertaine et provisoire dans de nombreux cas.

Dans cette situation, le présent inventeur a conduit des essais en vue d'identifier une substance susceptible d'être appliquée dans le traitement du psoriasis et plus généralement dans le traitement des dermatoses et de toute affection cutanée se manifestant ou provoquée par des formations kératosiques et/ou squameuses anormales du derme, qu'elles soient aiguës ou chroniques. De façon surprenante et inattendue, il a été trouvé que l'administration d'une composition à base de lécithine à un patient souffrant d'une telle affection conduit à d'excellents résultats, ce qui a abouti à la réalisation de la présente invention.

C'est pourquoi la présente invention a pour objectif de proposer une composition utile pour le traitement curatif des états provoqués ou se manifestant par des formations kératosiques et/ou squameuses anormales du derme, telles que celles qui se rencontrent dans les dermatoses récentes ou chroniques, et en particulier dans les différentes formes du psoriasis. Une action prophylactique est également recherchée.

De manière inattendue il a été trouvé que des compositions à base de lécithine permettaient de préparer un tel médicament. L'invention réside donc dans l'emploi de la lécithine ou d'un extrait riche en lécithine pour la préparation d'un médicament efficace dans la prévention et le traitement des dermatoses et autres affections récentes ou chroniques du derme, qui ne présente pas d'effet secondaire indésirable et dont la forme soit commode d'emploi pour le patient Les compositions thérapeutiques sont également objet de la présente invention.

La lécithine est un émulsifiant naturel connu dé longue date, abondant dans le jaune d'oeuf et le système nerveux. On la considère comme une substance indispensable au bon fonctionnement du système cardio-vasculaire, du cerveau, du système nerveux, du foie et de nombreux autres organes.

Selon un usage commun, on appelle "lécithine" un complexe composé principalement des phospholipides naturels (ou phosphoglycérides), à savoir essentiellement la phosphatidylcholine, le phosphatidylinositol, la phosphatidyléthanolamine, la phosphatidyl sérine auxquels s'ajoute la sphingomyéline.

Ces phosphoglycérides peuvent en outre être estérifiés sur la fonction alcool primaire libre par des acides gras, saturés ou insaturés. Ces acides gras peuvent notamment être des acides gras polyinsaturés du type oméga3, en particulier l'acide docosahexanoïque (DHA), l'acide eicosapentanoïque (EPA), l'acide docosapentanoïque (DPA). Il peut aussi s'agir d'un acide gras de type alkyl-glycérol. Le profil de répartition de ces différents phospholipides et de leurs dérivés estérifiés est variable d'un organisme à l'autre.

La lécithine peut être extraite des végétaux tels que la fève du soja, ou des animaux en particulier des poissons. Les méthodes d'extraction sont connues en soit. Elle est usuellement employée en alimentation animale et comme complément nutritionnel en diététique humaine.

Cependant son action en tant que médicament sur les affections cutanée était totalement inconnue. L'effet inattendu de la lécithine a été mis en évidence pour la première fois par le présent inventeur. Les lécithines marines, c'est-à-dire extraites des organismes marins, se sont avérées particulièrement intéressantes pour la mise en oeuvre de la présente invention.

Un premier objet de l'invention est l'utilisation de la lécithine dans une composition destinée à la fabrication d'un médicament utile dans la prévention et le traitement des états provoqués ou se manifestant par des formations squameuses et/ou kératosiques anormales du derme.

En particulier, ladite utilisation s'applique aux états choisis parmi le psoriasis habituel en points, en gouttes, nummulaires, en plaques, le psoriasis érythrodermique, le psoriasis arthropathique, le psoriasis pustuleux, psoriasis de l'enfant, le parapsoriasis, les dermatoses aiguës ou chroniques telles que les dermatoses ichtyosiques et kératosiques dont la kératose palmo-plantaire.

Selon un aspect particulier de l'invention, l'extrait comprend la lécithine dans une quantité de 10% à 50% en poids, dans une phase grasse. De préférence, la lécithine est présente à hauteur de 20% en poids dans l'extrait. L'extrait de lécithine se présente comme une huile liquide pouvant aller du jaune au brun selon son origine, et prenant une teinte brune orangée translucide lorsqu'elle est extraite du poisson. Elle est sensible à l'oxydation. Son acidité oléique est de 0,1%, son indice d'iode de 170 et son indice de peroxyde maximum de 5 meq/kg. Elle ne présente aucune toxicité aux doses d'emploi, chez l'animal comme chez l'humain.

La lécithine comprend les phospholipides suivants, en poids rapporté au poids total des phospholipides de la lécithine:
- de 10% à 75% de phosphatidylcholine, de préférence 45%,
- de 10% à 30% de phosphatidylinositol, de préférence 16%,
- de 5% à 30% de phosphatidyléthanolamine, de préférence 20%,
- de 5% à 20% de phosphatidylsérine, de préférence 5%, et
- de 5% à 30% de sphingomyéline, de préférence.5%.

Selon un aspect particulier de la présente invention, la lécithine comprend les phospholipides suivants, en poids rapporté au poids total des phospholipides de la lécithine :
- 45% de phosphatidylcholine,
- 16% de phosphatidylinositol,
- 20% de phosphatidyléthanolamine,
- 10% de phosphatidylsérine, et
- 5% de sphingomyéline.

Selon une caractéristique avantageuse de la présente invention, lesdits phospholipides sont estérifiés par des acides gras polyinsaturés du type oméga3. En effet, une fonction alcool primaire est présente dans les phospholipides cités plus haut, qui peut donc participer à une réaction d'estérification avec un acide, les acides gras polyinsaturés du type oméga3 étant préférés.

Selon un aspect préféré de la présente invention, lesdits phospholipides sont estérifiés par les acides gras du type oméga3 suivants, dans les proportions exprimées en poids rapporté au poids total desdits acides gras:
- par l'acide docosahexanoïque (DHA) à raison de 15% à 85%,
- par l'acide eicosapentanoïque (EPA), à raison de 5% à 35%,
- par l'acide docisapentanoïque (DPA), à raison de 0,5% à 5%,
- par un acide gras de type alkyl-glycérol, à raison de 5% à 30%.

De manière générale, les phospholipides sont majoritairement estérifiés par le DHA.

Selon un aspect particulier de l'utilisation conforme à l'invention, lesdits phospholipides sont estérifiés, par les acides gras suivants présents dans des proportions exprimées en poids rapporté au poids total desdits acides gras :
- par l'acide docosahexanoïque à raison d'au moins 17%,
- par l'acide eicosapentanoïque à raison d'au moins 14%,
- par l'acide docosapentanoïque à raison d'au moins 0,5%,
- par l'acide gras de type alkyl-glycérol à raison d'au moins 5%.

Selon une caractéristique intéressante de l'utilisation conforme à l'invention, la lécithine est extraite d'un organisme marin choisi parmi les poissons, les crevettes, le krill, le zooplancton, les algues, le phytoplancton ou d'un mélange de ceux-ci. On la qualifie alors de "lécithine marine". Son intérêt particulier réside dans le fait que ses phospholipides, notamment la phosphatidylcholine, sont naturellement estérifiés par des acides gras de type oméga3, et essentiellement par le DHA et l'EPA. Par extension on peut envisager d'utiliser des lécithines d'oeuf enrichies artificiellement en acides gras.

Les poissons sont de préférence originaires des mers froides, tout comme le krill qui est un plancton des mers froides formé de petits crustacés (essentiellement *Euphausia superba*) transparents, qui constitue la nourriture principale des baleines bleues.

Selon un mode préféré de réalisation de l'invention à l'aide d'un extrait de lécithine, ladite phase grasse est une huile de poisson, de préférence une huile de poisson riche en oméga3. De manière alternative, la lécithine utilisée pour l'utilisation selon l'invention peut également se présenter sous forme purifiée en poudre.

Finalement, ledit médicament peut avantageusement contenir la lécithine dans une quantité de 5% à 50%, de préférence de 10% à 30% en poids. Le médicament peut être utilisé selon une posologie permettant l'apport d'une dose efficace, se situant entre 0,4 g et 2 g de lécithine par jour. La forme orale est préférée, car la prise induit très peu de contrainte, ce qui permet une bonne tolérance des patients pour des cures d'une durée de plusieurs mois.

L'invention concerne une composition thérapeutique utile dans la prévention et le traitement des états provoqués ou se manifestant par des formations squameuses ou kératosiques anormales du derme, contenant de la lécithine ou un extrait de lécithine à titre de principe actif. En particulier, la composition selon l'invention est indiquée dans la prévention et le traitement de pathologies telles que le psoriasis habituel en points, en gouttes, nummulaires, en plaques, le psoriasis érythrodermique, le psoriasis arthropathique, le psoriasis pustuleux, psoriasis de l'enfant, le parapsoriasis, les dermatoses aiguës ou chroniques.

Selon une caractéristique intéressante, dans la composition thérapeutique selon l'invention, la lécithine comprend les phospholipides suivants, en poids rapporté au poids total des phospholipides de la lécithine:
- de 10% à 75% de phosphatidylcholine, de préférence 45%,
- de 10% à 30% de phosphatidylinositol, de préférence 16%,
- de 5% à 30% de phosphatidyléthanolamine, de préférence 20%,
- de 5% à 20% de phosphatidylsérine, de préférence 10%, et
- de 5% à 30% de sphingomyéline, de préférence 5%.

De préférence, la lécithine comprend les phospholipides suivants, en poids rapporté au poids total des phospholipides de la lécithine:
- 45% de phosphatidylcholine,
- 16% de phosphatidylinositol,
- 20% de phosphatidyléthanolamine,
- 10% de phosphatidylsérine, et
- 5% de sphingomyéline.

Selon une autre caractéristique intéressante, dans la composition thérapeutique selon l'invention, lesdits phospholipides sont estérifiés par des acides gras polyinsaturés du type oméga3.

De préférence, lesdits phospholipides sont estérifiés par, en poids rapporté au poids total desdits acides gras:
- l'acide docosahexanoïque à raison de 15% à 85%,
- l'acide eicosapentanoïque à raison de 5% à 35%,
- l'acide docosapentanoïque à raison de 0,5% à 5%,
- l'acide gras de type alkyl-glycérol à raison de 5% à 30%.

De manière encore préférée, lesdits phospholipides sont estérifiés par, en poids rapporté au poids total desdits acides gras:
- l'acide docosahexanoïque a raison d'au moins 17%,
- l'acide eicosapentanoïque a raison d'au moins 14%,
- l'acide docosapentanoïque à raison d'au moins 0,5%,
- l'acide gras de type alkyl-glycérol à raison d'au moins 5%.

Selon un mode de réalisation particulier de l'invention, la composition thérapeutique selon l'invention contient de la lécithine extraite d'un organisme marin choisi parmi les poissons, les crevettes, le krill, le zooplancton, les algues, le phytoplancton, ou d'un mélange de ceux-ci.

Selon un autre mode particulier de réalisation de la composition thérapeutique selon l'invention, la lécithine est apportée sous forme d'extrait dans une phase grasse, ladite phase grasse étant une huile de poisson, de préférence une huile de poisson riche en oméga3.

Le médicament préparé à l'aide de la composition comprenant de la lécithine peut se trouver sous une forme administrable par voie orale, par voie rectale ou par voie lymphatique directe. L'extrait huileux, comme les formes purifiées, peuvent être conditionnés en cellules. Il est également possible de présenter la composition selon l'invention sous toute forme telle que capsules, comprimés, suppositoires, ovules, crème, lotion, lait, pommade, gel ou poudre.

Si nécessaire, le médicament peut comprendre en outre un excipient convenant à une administration par voie orale, par voie rectale ou par voie lymphatique directe. Ce sera le cas notamment des compositions appliquées sur la peau. Un tel excipient peut être judicieusement choisi par l'homme du métier parmi des excipients d'usage thérapeutique neutres vis-à-vis des actifs, en fonction de la voie d'administration retenue et de la consistance finale souhaitée. Par exemple, on peut utiliser l'eau, le propylène glycol, le butylène glycol, les diglycérols éthoxylés ou propoxylés.

La lécithine ou l'extrait de lécithine utilisé conformément à la présente invention peut bien entendu être accompagnés d'autres composants tels que des additifs ou des supports de vectorisation, selon les techniques connues de l'homme de l'art. Les additifs sont par exemple ceux nécessaires à la formulation convenable du médicament contenant la lécithine tels que des épaississants, des tensio-actifs, des anti-oxydants, des colorants, des conservateurs, des parfums. La vectorisation pourra être réalisée par solubilisation dans des liposomes, adsorption sur des polymères organiques poudreux, sur des supports minéraux comme le talc ou les bentonites, ou à l'aide de tout autre vecteur pharmaceutiquement acceptable.

L'objet de la présente invention, ainsi que ses modes de mise en oeuvre possibles et les avantages qui y sont liés, apparaîtront plus clairement à la lecture des exemples suivants donnés à titre illustratif.

### EXEMPLE 1

Caractérisation d'un extrait de lécithine marine dans une phase grasse

Un extrait de lécithine marine utilisable .selon l'invention est par exemple l'extrait, commercialisé par le laboratoire Phytobiolab sous la marque OEMINE MER™". Il est préconisé comme complément alimentaire, avec pour indication principale l'action préventive sur le système cardio-vasculaire et le système nerveux, qui sont les indications habituelles des oméga 3. L'indication dermatologique n'était pas connue jusqu'à présent

L'extrait contient 20% de lécithine marine d'un poisson sauvage des mers froides. La lécithine marine contient les phospholipides suivants : environ 45% de phosphatidylcholine estérifiée par le DHA, 20% de phosphatidyléthanolamine,16% de phosphatidylinositol, 5% de phosphatidylsérine et 5% de sphingomyéline.

Les phospholipides estérifiés sont associés à l'acide docosahexanoïque (DHA) à raison de 60 %, à l'acide eicosapentanoïque (EPA) à raison de 30 %. L'acide docosapentanoïque (DPA) est présent à un taux d'environ 1%, et l'acide gras de type alkyl-glycérol à un taux d'environ 5 %. La phase grasse est constituée d'huile de poisson riche à 34% en OMEGA 3.

L'extrait est conditionné en doses de 500 mg sous forme de capsules de gélatine selon les techniques connues de la galénique. Aucun additif ni excipient n'est ajouté. Les tocophérols naturellement présents dans l'huile jouent le rôle d'anti-oxydant. Chaque capsule contient une dose utile de 100 mg de lécithine.

### EXEMPLE 2

L'effet de la prise régulière de lécithine marine sur le psoriasis a été mis en évidence à l'occasion d'une étude dont les résultats sont présentés dans les Tableaux 1 et 2.

Les tests ont été réalisés sur un échantillon de 20 personnes, 10 hommes et 10 femmes, âgés de 17 à 71 ans. Toutes les formes de psoriasis y sont représentées, psoriasis en plaque, psoriasis du cuir chevelu, psoriasis en gouttes, psoriasis érythrodermique et psoriasis inversé.

Le traitement a consisté en 4 capsules par jour en 2 prises, soit une dose quotidienne de lécithine de 0,4 g.

Toutes les personnes ayant reçu le traitement ont vu leurs plaques régresser peu à peu en 2 à 4 mois et complètement cicatriser en 4 à 6 mois. Aucun traitement habituel du psoriasis n'a été utilisé durant cette étude de telle sorte que le résultat obtenu ne peut être mis que sur le compte de la lécithine.

Légende des tableaux :
Légère amélioration: +;
Amélioration significative: ++;
Disparition totale des signes cliniques: +++.

### EXEMPLE 2a

Le Tableau 1 présente les résultats obtenus dans le traitement de 6 patients atteints de psoriasis apparu depuis moins de 6 ans.

**TABLEAU 1**

| durée de l'affection | observations cliniques après traitement durant | | | | |
|---|---|---|---|---|---|
| | 1 mois | 2 mois | 3 mois | 4 mois | 6 mois |
| 3 ans | ++ | | | +++ | |
| 5 ans | + | | | ++ | +++ |
| 1 mois | | ++ | | +++ | |
| 6 mois | + | +++ | | | |
| 4 ans | | +++ | | | |
| 2 ans | | | | + | +++ |

### EXEMPLE 2b

Le Tableau 2 présente les résultats obtenus dans le traitement de 14 patient atteints de psoriasis depuis plus de 5 ans.

**TABLEAU 2**

| durée de l'affection | observations cliniques après traitement durant | | | | |
|---|---|---|---|---|---|
| | 1 mois | 2 mois | 3 mois | 4 mois | 6 mois |
| 47 ans | | + | | +++ | |
| 28 ans | | | | | ++ |
| 54 ans | | | | | +++ |
| 15 ans | | ++ | | +++ | |
| 10 ans | | ++ | | | |
| 24 ans | | | + | | ++ |
| 20 ans | | | | | +++ |
| 17 ans | | ++ | | +++ | |
| 13 ans | | | ++ | | |
| 20 ans | | + | | | ++ |
| 6 ans | + | ++ | | | |
| 10 ans | | + | | ++ | |
| 18 ans | + | + | | + | |
| 25 ans | | + | | | +++ |

Sur un total de 14 patients suivis, on note dès le 3ème mois de traitement, une amélioration pour tous les patients. L'amélioration est légère chez 64% des patients (9/14) et nette chez 36% d'entre eux (5/15).

Après 6 mois, on totalise 43% de guérisons complètes (6 patients), dont 3 après 4 mois de traitement seulement. Chez tous les autres patients, soit 57%, l'amélioration de l'état est nette au bout de 6 mois. Dès le 3ème mois de traitement, on observe une amélioration nette ou une disparition totale des plaques pour 67% es malades (4/6) et une légère amélioration de l'état clinique pour 33% des patients (2/6). Après 6 mois, on totalise 100% de guérisons complètes.

### EXEMPLE 3

L'effet bénéfique de l'utilisation de la lécithine selon l'invention est illustré par les études de cas cliniques ci-après.

Sauf indication contraire, suite à la première consultation, tous les patients ont reçu un traitement consistant en 4 capsules de 0,5 g à 20% de lécithine par jour en 2 prises, soit une dose quotidienne de lécithine de 0,4 g.

### Observation 1

Patiente de 59 ans. Psoriasis depuis l'âge de 12 ans (47 ans) sans aucune guérison sauf deux rémissions, l'une pendant la grossesse et l'autre pendant une cure de puvathérapie 4 ans auparavant, mais avec une rémission partielle suivie d'une récidive immédiate.
- 1ère consultation : lésions étendues, en ceinture, aux coudes et à l'orée du cuir chevelu faites de larges plaques squameuses peu prurigineuses.
- 2eme consultation ( 2 mois après) : les plaques vont mieux.
- 3eme consultation ( 4 mois après) : toutes les plaques ont disparu totalement.

### Observation 2

Patient de 52 ans. Psoriasis depuis 28 ans, coude, cuir chevelu et organes génitaux. Suivi depuis 20 ans sans rémission avec des soins locaux et des huiles de poisson "biosaumon" depuis 1988.
- 1ere consultation : psoriasis inflammatoire et squameux des coudes, prurigineux au cuir chevelu.
- 2eme consultation ( 6 mois après) : très nette amélioration des plaques.

### Observation 3

Patient de 43 ans. Psoriasis apparu il y a 4 ans, traité par corticoïdes, puvathérapie. Sans rémission. Localisé aux coudes, genoux, région médio-thoracique, cuir chevelu.
- 1ère consultation : psoriasis en train de s'étendre, très inflammatoire, localisé au thorax coude genoux cuir chevelu.
- 2eme consultation (1 mois après) : très nette amélioration des plaques.
- au bout de 4 mois de traitement, toutes les plaques ont totalement disparues.

### Observation 4

Patiente de 66 ans. Psoriasis ancien depuis l'âge de 12 ans (54 ans) généralisé, mais pas au cuir chevelu. Suivie à l'hôpital St louis, à Paris. Elle dit avoir tout essayé sans résultats.
- 1ère consultation: lésions très étendues, en goutte, peu squameuse, sans prurit et un peu irrité. Localisations surtout aux membres inférieurs, aux bras et aux fesses.
- 2eme consultation (6 mois après) : toutes les plaques ont disparu.

### Observation 5

Patiente de 39 ans. Psoriasis apparu à la suite d'une angine streptococcique il y a 2 ans, en gouttes, traité par corticoïdes, sans rémission, associé à un rhumatisme.
- 1ere consultation: psoriasis en train de s'étendre, très inflammatoire, en gouttes, localisé au thorax, coudes, genoux, poitrine et au cuir chevelu, associé à un rhumatisme localisé aux inter-phalangiennes distales des deux mains, aux coudes et chevilles.
- 2eme consultation (1 mois après) : nette amélioration des plaques. Il en reste quelques unes mais le rhumatisme persiste.
- 3eme consultation (4 mois après) ; il ne reste que quelques plaques sur le thorax. Les douleurs des genoux vont mieux.
- 4eme consultation (6 mois après) : la peau est complètement guérie, le rhumatisme persiste.

### Observation 6

Patient de 32 ans. Psoriasis ancien, en goutte depuis l'âge de 17 ans (15 ans) apparu brutalement pendant l'été. Il dit avoir déjà tout essayé, puvathérapie, corticothérapie, sans succès.
- 1er consultation: lésions en gouttes, sur tout le thorax et les jambes, placards confluant dans la région médiothoracique. Aux, alentours d'une centaine d'éléments rien que sur la partie antérieure et le double sur la partie postérieure du thorax.
- 2eme consultation (2 mois après) : nette amélioration les lésions blanchissent sur le dos on compte moitié moins de lésions. Les plaques confluantes du thorax sont guéries, celles qui ne le sont pas totalement blanchissent de l'intérieur vers la périphérie.
- 3eme consultation (4 mois après) : disparition totale de toutes les plaques. Il avait essayé d'arrêter 10 jour le traitement et cela revenait un peu aux jambes. Il ne prends plus que de lui même deux capsules par jour.

### Observation 7

Patient de 17 ans. Psoriasis depuis 10 ans, généralisé, en goutte, survenu suite à la perte de son chien.
- 1er consultation : lésions en gouttes au thorax.
- 2eme consultation (2 mois après) : les plaques sont blanchies.

### Observation 8

Patiente de 30 ans. Psoriasis ancien, depuis l'âge de 6 ans (24 ans) aux zones de frottement, avec un rhumatisme psoriasique sévère, traité par corticoïdes locaux et vitamine D sans rémission notable.
- 1 ere consultation: plaques très épaisses lichénifiées au dos de la main, aux coude, et avec placards erythémato-squameux disséminés à la jambe, squames épaisses, associées à rhumatisme psoriasique, des inter-phalangiennes des deux mains, des pieds, et des chevilles très enflammées.
- 2eme consultation (3 mois après) : légère amélioration des plaques surtout aux jambes. Elle signale qu'elle avait des troubles digestifs qui ont disparu. Les rhumatismes vont mieux.
- 3eme consultation (6 mois après) : très nette amélioration de toutes les plaques. Celles des jambes sont les plus améliorées et sont en voie de cicatrisation. Les autres plus lichenifiées notamment à la main et au coude se sont beaucoup désépaissies.

### Observation 9

Patient de 62 ans. Psoriasis du dos depuis 20 ans.

Les plaques ont totalement disparu au bout de 6 mois de traitements.

### Observation 10

Patient de 47 ans. Psoriasis ancien depuis l'âge de 30 ans (17 ans) localisé aux coude, plis, nombril. Puvathérapie pendant 5 ans sans rémission totale.
- 1 ere consultation : lésions coude, genoux, nombril. Peau épaisse blanche sur fond rosé, squames épaisses, rugueuses, infiltrées.
- 2eme consultation (2 mois après) : bonne amélioration. Peau deux fois moins épaisse, disparition des épaisseurs blanchâtres et peau rosée moins infiltrée. La périphérie des lésions est moins irritée et commence à guérir. Élution centripète vers la guérison.
- 3eme consultation (4 mois après) : tout a cicatrisé.

### Observation 11

Patiente de 50 ans. Psoriasis ancien depuis 13 ans au cuir chevelu et derrière les oreilles. Traité par corticoïdes avec récidives constantes.
- 1ere consultation : lésions derrière les oreilles et au cuir chevelu peu étendu mais prurigineux.
- 2eme consultation (3 mois après) : bonne amélioration. Peau deux fois moins épaisses, les lésions ont disparu mais il persiste des troubles digestifs.

### Observation 12

Patiente de 54 ans. Psoriasis ancien depuis 20 ans en plaque et inversé. Sans amélioration malgré corticoïdes puis soignée depuis 4 ans par huile de poissons avec petite amélioration.
- 1ere consultation : lésions du pubis, du pli inter-fessier avec prurit anal, de la jambe. Larges plaques squameuse, très prurigineuses et irritées avec excoriations.
- 2eme consultation (2 mois après) : bonne amélioration. Peau deux fois moins épaisses, légère amélioration surtout au niveau de la plaque de la jambe.
- 3eme consultation (6 mois après): les lésions sont cicatrisées.

### Observation 13

Patient de 41 ans. Psoriasis depuis 1 mois généralisé après des soucis pour un examen. Tout le thorax et les jambes sans amélioration malgré corticoïdes.
- 1ere consultation : lésions généralisées à tout le thorax et à la jambe droite, peu épais mais enflammé.
- 2eme consultation (2 mois après) : les plaques sont en voie de guérison. Elles ont séché. La jambe est à moitié cicatrisée.
- 3eme consultation (4 mois après) : tout a cicatrisé.

### Observation 14

Patiente de 71 ans. Psoriasis généralisé depuis 6 mois, sur tout le corps avec eczématisation et surinfection des lésions par du staphylocoque doré, traité sans résultats par corticoïdes locaux et généraux. Certaines plaques notamment aux membres inférieurs ont tendance à suinter. Les lésions s'associent à une lymphangite du membre intérieur droit.
- 1ere consultation : lésions très étendues, la patiente est en pleur, ne supporte plus les brûlures. Traitement par antibiothérapie associée à une cure de 2 capsules de lécithine matin et soir.
- 2eme consultation (1 mois après) : nette amélioration. In'y a plus de suintements ni d'oedème, les plaques commencent à blanchir de l'intérieur vers la périphérie. Elles ne s'étendent plus. La patiente est moins fatiguée. On stoppe l'antibiothérapie et on continue le traitement par 4 capsules par jour.
- 3eme consultation (2 mois après) : disparition totale de toutes les plaques. Peau totalement cicatrisée sans aucune marque. Il reste quelques zones un peu enflammées.

### Observation 15

Patiente de 44 ans. Psoriasis apparu il y a 6 ans, après une séparation, traité par corticoïdes, puvathérapie.
- 1 ere consultation : forme érythrodermique généralisé, squames épaisses surtout très inflammatoire, peau rôtie, sans intervalle de peau saine, localisé à tout le tronc, en ceinture des hanches jusqu'aux cotes, et des coudes jusqu'aux poignets très étendu, inflammatoire, avec sensation de brûlure.
- 2eme consultation (1 mois après) : mêmes localisations mais peu de squames. La peau est moins irritée.
- 3eme consultation (2 mois après) : disparition totale des plaques. Restent seulement quelques petites lésions en périphérie des plaques. Peau totalement cicatrisée sans aucune marque, souple et correctement hydratée.

### Observation 16

Patient de 53 ans. Psoriasis depuis plus de 10 ans, généralisé surtout aux fesses et aux coudes.
- 1ere consultation : lésions étendues surtout deux grosses plaques très épaisses aux coudes.
- 2eme consultation (2 mois après) : les plaques vont mieux, sont bien moins épaisses.
- 3eme consultation (4 mois après) : toutes les plaques ont pratiquement disparu.

### Observation 17

Patient de 30 ans: psoriasis depuis 4 ans en plaques.
- 1 ere consultation : lésions peu étendues, coudes, crète tibiale, peu épaisses.
- 2eme consultation : les plaques ont disparu après deux mois de traitement.

### Observation 18

Patiente de 16 ans. Psoriasis depuis 2 ans au cuir chevelu traité par corticoïdes sans succès.
- 1 ere consultation : lésions très étendues, sur tout le cuir chevelu, avec de larges plaques squameuses.
- 2eme consultation (4 mois après) : les plaques vont mieux, elles on arrêté de s'étendre. On diminue la posologie de moitié.
- 3eme consultation (6 mois après) : les plaques ont disparu totalement.

### Observation 19

Patiente de 32 ans. Psoriasis ancien depuis l'âge de 14 ans (18 ans) généralisé, y compris au cuir chevelu avec des lésions engainantes, apparues après une forte angine. Tous les traitements ont été essayé sans succès.
- 1ere consultation : lésions très étendues, peau épaisse infiltrée, peu squameuse, excoriée. Localisations surtout aux membres inférieurs, sans intervalle de peau saine.
- 2eme consultation (1 mois après) : petit début d'amélioration. Peau moins infiltrée.
- 3eme consultation (2 mois après) : des zones saines commencent à apparaître, la peau est plus souple, moins irritée, moins prurigineuse. Surtout, la patiente note une augmentation de sa sudation alors qu'auparavant elle ne suait pas bien, notamment en été.
- 4eme consultation (4 mois après) : évolution remarquable. Il ne reste que quelques excoriations et quelques zones encore un peu infiltrée, mais plus de squames.

### Observation 20

Patient de 70 ans. Psoriasis ancien depuis 25 ans qui est en train de se généraliser, malgré prise de Soriatane™ qu'il ne supporte plus. Traité d'abord par des soins locaux mais sans grande amélioration; puis avec de l'huile de poisson, mais également sans succès majeur.
- 1ere consultation : lésions très étendues, coudes genoux, larges plaques aux fesses et sur la région hépatique, squameuses, sans prurit et un peu irrité. Localisation également au cuir chevelu.
- 2eme consultation (2 mois après) : les plaques vont mieux, elles on arrêté de s'étendre.
- 3eme consultation (6 mois après) : les plaques ont disparu.

## Revendications

1. Utilisation de la lécithine ou d'un extrait riche en lécithine pour la fabrication d'un médicament pour administration par voie orale, rectale ou lymphatique directe pour la prévention et le traitement des états provoqués ou se manifestant par des formations squameuses ou kératosiques anormales du derme dans laquelle :
- la lécithine comprend les phospholipides suivants, en poids rapporté au poids total des phospholipides de la lécithine :
- de 10 à 75% de phosphatidylcholine, de préférence 45%,
- de 10 à 30% de phosphatidylinositol, de préférence 16%,
- de 5 à 30% de phosphatidyléthanolamine, de préférence 20%,
- de 5 à 20% de phosphatidylsérine, de préférence 10%, et
- de 5 à 30% de sphingomyéline, de préférence 5%.
- lesdits phospholipides étant estérifiés par des acides gras polyinsaturés du type oméga 3.

2. Utilisation selon la revendication 1, **caractérisée en ce que** lesdits états sont choisis parmi le psoriasis habituel en points, en gouttes, nummulaires, en plaques, le psoriasis érythrodermique, le psoriasis arthropathique, le psoriasis pustuleux, psoriasis de l'enfant, le parapsoriasis, les dermatoses aiguës ou chroniques.

3. Utilisation selon la revendication 1 ou 2, **caractérisée en ce que** ledit extrait comprend la lécithine dans une quantité de 10% à 50% en poids, de préférence 20%, en poids, dans une phase grasse.

4. Utilisation selon l'une des revendications 1 à 3, **caractérisée en ce que** la lécithine comprend les phospholipides suivants, en poids rapporté au poids total des phospholipides de la lécithine :
- 45% de phosphatidylcholine,
- 16% de phosphatidylinositol,
- 20% de phosphatidyléthanolamine,
- 10% de phosphatidylsérine, et
- 5% de sphingomyéline.

5. Utilisation selon la revendication 4, **caractérisée en ce que** lesdits phospholipides sont estérifiés par, en poids rapporté au poids total desdits acides gras :
- l'acide docosahexanoïque à raison de 15% à 85%
- l'acide eicosapentanôique à raison de 5% à 35%
- l'acide docosapentanoïque à raison de 0,5% à 5%
- l'acide gras de type alkyl-glycérol à raison de 5% à 30%.

6. Utilisation selon l'une des revendications 4 ou 5, **caractérisée en ce que** lesdits phospholipides sont estérifiés par, en poids rapporté au poids total desdits acides gras :
- l'acide docosahexanoïque à raison d'au moins 17%
- l'acide eicosapentanoïque à raison d'au moins 14%
- l'acide docosapentanoïque à raison d'au moins 0,5%
- l'acide gras de type alkyl-glycérol à raison d'au moins 5%.

7. Utilisation selon l'une des revendications 1 à 6, **caractérisée en ce que** la lécithine est extraite d'un organisme marin choisi parmi les poissons, les crevettes, le krill, le zooplancton, les algues, le phytoplancton ou d'un mélange de ceux-ci.

8. Utilisation selon l'une des revendications 1 à 7, **caractérisée en ce que** dans l'extrait de lécithine, ladite phase grasse est une huile de poisson, de préférence une huile de poisson riche en oméga 3.

9. Utilisation selon l'une des revendications 1 à 8, **caractérisée en ce que** ledit médicament comprenant la lécithine ou extrait riche en lécithine est administrée par voie orale.

10. Composition thérapeutique administrable par voie orale, rectale ou lymphatique directe pour utilisation dans la prévention et le traitement des états provoqués ou se manifestant par des formations squameuses ou kératosiques anormales du derme, contenant de la lécithine ou un extrait de lécithine à titre de principe actif, les phospholipides suivants, en poids rapporté au poids total des phospholipides de la lécithine :
- de 10 à 75% de phosphatidylcholine, de préférence 45%,
- de 10 à 30% de phosphatidylinositol, de préférence 16%,
- de 5 à 30% de phosphatidyléthanolamine, de préférence 20%,
- de 5 à 20% de phosphatidylsérine, de préférence 10%, et
- de 5 à 30% de sphingomyéline, de préférence 5%, et
lesdits phospholipides étant estérifiés par des acides gras polyinsaturés du type oméga3.

11. Composition thérapeutique pour utilisation selon la revendication 10, dans laquelle la lécithine comprend les phospholipides suivants, en poids rapporté au poids total des phospholipides de la lécithine :
- 45% de phosphatidylcholine,
- 16% de phosphatidylinositol,
- 20% de phosphatidyléthanolamine,
- 10% de phosphatidylsérine, et
- 5% de sphingomyéline.

12. Composition thérapeutique pour utilisation selon l'une des revendications 10 ou 11 dans laquelle lesdits phospholipides sont estérifiés par, en poids rapporté au poids total desdits acides gras :
- l'acide docosahexanoïque à raison de 15% à 85%
- l'acide eicosapentanoïque à raison de 5% à 35%
- l'acide docosapentanoïque à raison de 0,5% à 5%
- l'acide gras de type alkyl-glycérol à raison de 5% à 30%.

13. Composition thérapeutique pour utilisation selon l'une des revendications 10 à 12 dans laquelle lesdits phospholipides sont estérifiés par, en poids rapporté au poids total desdits acides gras :
- l'acide docosahexanoïque à raison d'au moins 17%
- l'acide eicosapentanoïque à raison d'au moins 14%
- l'acide docosapentanoïque à raison d'au moins 0,5%
- l'acide gras de type alkyl-glycérol à raison d'au moins 5%.

14. Composition thérapeutique pour utilisation selon l'une des revendications 10 à 13 dans laquelle la lécithine est extraite d'un organisme marin choisi parmi les poissons, les crevettes, le krill, le zooplancton, les algues, le phytoplancton, ou d'un mélange de ceux-ci.

15. Composition thérapeutique pour utilisation selon l'une des revendications 10 à 14 dans laquelle la lécithine est apportée sous forme d'extrait dans une phase grasse, ladite phase grasse étant une huile de poisson, de préférence une huile de poisson riche en oméga 3.

16. Composition thérapeutique pour utilisation selon l'une des revendications 10 à 15, **caractérisée en ce qu'**elle est administrable par voie orale.

## Claims

1. The use of lecithin or an extract rich in lecithin for the manufacturing a medicine administrable by an oral, rectal or direct lymphatic route, useful for prevention and treatment of conditions provoked by, or appearing in the form of scaly or keratosic formations of the derma wherein :
- said lecithin comprises the following phospholipids, by weight of total phospholipids of lecithin:
- from 10% to 75% phosphatidylcholine, preferably 45 %,
- from 10% to 30% phosphatidylinositol, preferably 16 %,
- from 5% to 30% phosphatidylethanolamine, preferably 20 %,
- from 5% to 20% phosphatidylserine, preferably 10 %, and
- from 5% to 30% sphingomyeline, preferably 5 %.
- said phospholipids being esterified by Omega 3-type polyunsaturated fatty acids.

2. The use according to claim 1, wherein said conditions are selected from the group consisting of common psoriasis, guttate psoriasis, nummular psoriasis, plaque psoriasis, erytrhodermic psoriasis, psoriatic arthritis, pustular psoriasis, child psoriasis, parapsoriasis, and chronic and acute dermatitis.

3. The use according to claim 1 or 2, wherein said lecithin rich extract comprises lecithin in an amount of 10-50% by weight, preferably 20% by weight, in a fatty phase.

4. The use according to claims from 1 to 3, wherein said lecithin comprises the following phospholipids, by weight of total phospholipids of lecithin:
- 45% phosphatidylcholine,
- 16% phosphatidylinositol,
- 20% phosphatidylethanolamine,
- 10% phosphatidylserine, and
- 5% sphingomyeline.

5. The use according to claim 4, wherein said phospholipids are esterified by fatty acids, in an amount by weight of total fatty acids:
- docosahexaenoic acid within a range of 15% to 85%,
- eicosapentaenoic acid within a range of 5% to 35%,
- docosapentaenoic acid within a range of 0.5% to 5%,
- a fatty acid of the alkyl glycerol type within a range of 5% to 30%.

6. The use according to claim 4 or 5, wherein said phospholipids are esterified by the fatty acids, in an amount by weight of total fatty acids:
- docosahexaenoic acid at least 17%,
- eicosapentaenoic acid at least 14%,
- docosapentaenoic acid at least 0.5%,
- the fatty acid of the alkyl glycerol type at least 5%.

7. The use according to one of the claims from 1 to 6, wherein said lecithin is lecithin extracted from a marine organism selected from the group consisting of fish shrimps, krill, zooplankton, algae, phytoplankton, and mixtures thereof.

8. The use according to one of the claims from 1 to 7, wherein said lecithin comprises a lecithin extract in a fatty phase, the said fatty phase being a fish oil, preferably an Omega-3 rich fish oil.

9. The use according to one of the claims from 1 to 8, wherein said medicine comprising lecithin or an extract rich in lecithin is administered orally.

10. A therapeutic composition administrable by an oral, rectal or direct lymphatic route, for the use in prevention and treatment of conditions provoked by, or appearing in the form of scaly or keratosic formations of the derma wherein said lecithin comprises the following phospholipids, by weight of total phospholipids of lecithin:
- from 10% to 75% phosphatidylcholine,
- from 10% to 30% phosphatidylinositol,
- from 5% to 30% phosphatidylethanolamine,
- from 5% to 20% phosphatidylserine,
- from 5% to 30% sphingomyeline, said phospholipids being esterified by Omega 3-type polyunsaturated fatty acids.

11. A therapeutic composition for the use according to claim 10, wherein said lecithin comprises the following phospholipids, by weight of total phospholipids of lecithin:
- 45% phosphatidylcholine,
- 16% phosphatidylinositol,
- 20% phosphatidylethanolamine,
- 10% phosphatidylserine, and
- 5% sphingomyeline.

12. A therapeutic composition for the use according to claim 10 and 11, wherein said phospholipids are esterified by fatty acids, in an amount by weight of total fatty acids:
- docosahexaenoic acid within a range of 15% to 85%,
- eicosapentaenoic acid within a range of 5% to 35%,
- docosapentaenoic acid within a range of 0.5% to 5%, and
- a fatty acid of the alkyl glycerol type within a range of 5% to 30%.

13. A therapeutic composition for the use according to claims from 10 to 12, wherein said phospholipids are esterified by the fatty acids, in an amount by weight of total fatty acids:
- docosahexaenoic acid at least 17%,
- eicosapentaenoic acid at least 14%,
- docosapentaenoic acid at least 0.5%,
- the fatty acid of the alkyl glycerol type at least 5%.

14. A therapeutic composition for the use according to one of the claims from 10 to 13, wherein said lecithin is lecithin extracted from a marine organism selected from the group consisting of fish shrimps, krill, zooplankton, algae, phytoplankton, and mixtures thereof.

15. A therapeutic composition for the use according to one of the claims from 9 to 13, wherein said lecithin comprises a lecithin extract in a fatty phase, the said fatty phase being a fish oil, preferably an Omega-3 rich fish oil.

16. A therapeutic composition for the use according to one of the claims from 10 to 15, wherein it is administered orally.

## Patentansprüche

1. Verwendung von Lecithin oder eines lecithinreichen Extraktes für die Herstellung eines Medikaments zur Verabreichung auf oralem, rektalem oder direktem lymphatischen Weg für die Prävention und die Behandlung der Zustände, die durch anormale squamöse oder keratotische Formationen der Dermis hervorgerufen werden oder sich **dadurch** manifestieren, wobei:
- das Lecithin die folgenden Phospholipide, in Gewicht bezogen auf das Gesamtgewicht der Phospholipide des Lecithins umfaßt:
- 10 bis 75 % Phosphatidylcholin, vorzugsweise 45 %,
- 10 bis 30 % Phosphatidylinositol, vorzugsweise 16 %,
- 5 bis 30 % Phosphatidylethanolamin, vorzugsweise 20 %,
- 5 bis 20 % Phosphatidylserin, vorzugsweise 10 %, und
- 5 bis 30 % Sphingomyelin, vorzugsweise 5 %,
- wobei die Phospholipide durch mehrfach ungesättigte Fettsäuren des Typs Omega-3 verestert werden.

2. Verwendung nach Anspruch 1, **dadurch gekennzeichnet, daß** die Zustände ausgewählt sind aus der üblichen punkförmigen, tropfenförmigen Psoriasis, Psoriasis nummularis, Plaque-Psoriasis, erythrodermalen Psoriasis, Psoriasis arthropathica, Psoriasis pustulosa, Psoriasis bei Kindern, Parapsoriasis, akuten oder chronischen Dermatosen.

3. Verwendung nach Anspruch 1 oder 2, **dadurch gekennzeichnet, daß** der Extrakt das Lecithin in einer Menge zwischen 10 Gew.-% und 50 Gew.-%, vorzugsweise 20 Gew.-%, in einer Fettphase enthält.

4. Verwendung nach einem der Ansprüche 1 bis 3, **dadurch gekennzeichnet, daß** das Lecithin die folgenden Phospholipide, in Gewicht bezogen auf das Gesamtgewicht der Phospholipide des Lecithins umfaßt:
- 45 % Phosphatidylcholin,
- 16 % Phosphatidylinositol,
- 20 % Phosphatidylethanolamin,
- 10 % Phosphatidylserin und
- 5 % Sphingomyelin.

5. Verwendung nach Anspruch 4, **dadurch gekennzeichnet, daß** die Phospholipide verestert werden mit, in Gewicht bezogen auf das Gesamtgewicht der Fettsäuren:
- Docosahexaensäure in einer Menge von 15 % bis 85 %,
- Eicosapentaensäure in einer Menge von 5 % bis 35 %,
- Docosapentaensäure in einer Menge von 0,5 % bis 5 %,
- Fettsäure vom Typ Alkyl-Glycerol in einer Menge von 5 % bis 30 %.

6. Verwendung nach einem der Ansprüche 4 oder 5, **dadurch gekennzeichnet, daß** die Phospholipide verestert werden mit, in Gewicht bezogen auf das Gesamtgewicht der Fettsäuren:
- Docosahexaensäure in einer Menge von wenigstens 17 %,
- Eicosapentaensäure in einer Menge von wenigstens 14 %,
- Docosapentaensäure in einer Menge von wenigstens 0,5 %,
- Fettsäure vom Typ Alkyl-Glycerol in einer Menge von wenigstens 5 %.

7. Verwendung nach einem der Ansprüche 1 bis 6, **dadurch gekennzeichnet, daß** das Lecithin aus einem Meeresorganismus gewonnen wird, der aus Fischen, Garnelen, Krill, Zooplankton, Algen, Phytoplankton oder einer Mischung aus diesen gewählt ist.

8. Verwendung nach einem der Ansprüche 1 bis 7, **dadurch gekennzeichnet, daß** in dem Lecithinextrakt die Fettphase ein Fischöl ist, vorzugsweise ein Fischöl, welches reich an Omega-3 ist.

9. Verwendung nach einem der Ansprüche 1 bis 8, **dadurch gekennzeichnet, daß** das Medikament, welches das Lecithin oder einen lecithinreichen Extrakt enthält, auf oralem Weg verabreicht wird.

10. Therapeutische, auf oralem, rektalem oder direktem lymphatischen Weg verabreichbare Zusammensetzung zur Verwendung bei der Prävention und der Behandlung der Zustände, die durch anormale squamöse oder keratotische Formationen der Dermis hervorgerufen werden oder sich **dadurch** manifestieren, enthaltend Lecithin oder einen Lecithinextrakt als Wirkstoff, die folgenden Phospholipide, in Gewicht bezogen auf das Gesamtgewicht der Phospholipide des Lecithins:
- 10 bis 75 % Phosphatidylcholin, vorzugsweise 45 %,
- 10 bis 30 % Phosphatidylinositol, vorzugsweise 16 %,
- 5 bis 30 % Phosphatidylethanolamin, vorzugsweise 20 %,
- 5 bis 20 % Phosphatidylserin, vorzugsweise 10 %, sowie
- 5 bis 30 % Sphingomyelin, vorzugsweise 5 %, und
- wobei die Phospholipide durch mehrfach ungesättigte Fettsäuren des Typs Omega-3 verestert werden.

11. Therapeutische Zusammensetzung zur Verwendung, nach Anspruch 10, wobei das Lecithin die folgenden Phospholipide, in Gewicht bezogen auf das Gesamtgewicht der Phospholipide des Lecithins umfaßt:
- 45 % Phosphatidylcholin,
- 16 % Phosphatidylinositol,
- 20 % Phosphatidylethanolamin,
- 10 % Phosphatidylserin und
- 5 % Sphingomyelin.

12. Therapeutische Zusammensetzung zur Verwendung, nach einem der Ansprüche 10 bis 11, wobei die Phospholipide verestert werden mit, in Gewicht bezogen auf das Gesamtgewicht der Fettsäuren:
- Docosahexaensäure in einer Menge von 15 % bis 85 %,
- Eicosapentaensäure in einer Menge von 5 % bis 35 %,
- Docosapentaensäure in einer Menge von 0,5 % bis 5 %,
- Fettsäure vom Typ Alkyl-Glycerol in einer Menge von 5 % bis 30 %.

13. Therapeutische Zusammensetzung zur Verwendung, nach einem der Ansprüche 10 bis 12, wobei die Phospholipide verestert werden mit, in Gewicht bezogen auf das Gesamtgewicht der Fettsäuren:
- Docosahexaensäure in einer Menge von wenigstens 17 %,
- Eicosapentaensäure in einer Menge von wenigstens 14 %,
- Docosapentaensäure in einer Menge von wenigstens 0,5 %,
- Fettsäure vom Typ Alkyl-Glycerol in einer Menge von wenigstens 5 %.

14. Therapeutische Zusammensetzung zur Verwendung, nach einem der Ansprüche 10 bis 13, wobei das Lecithin aus einem Meeresorganismus gewonnen wird, der aus Fischen, Garnelen, Krill, Zooplankton, Algen, Phytoplankton oder einer Mischung aus diesen gewählt ist.

15. Therapeutische Zusammensetzung zur Verwendung, nach einem der Ansprüche 10 bis 14, wobei das Lecithin in Extraktform in eine Fettphase eingebracht wird, wobei die Fettphase ein Fischöl ist, vorzugsweise ein Fischöl, welches reich an Omega-3 ist.

16. Therapeutische Zusammensetzung zur Verwendung, nach einem der Ansprüche 10 bis 15, **dadurch gekennzeichnet, daß** sie auf oralem Weg verabreichbar ist.
